# EUROPEAN PATENT APPLICATION

(11) **EP 2 888 951 A1**
(43) Date of publication of application: **01.07.2015**
(21) Application number: 13199586.2
(22) Date of filing: 24.12.2013
(51) Int. Cl.: A24B 15/16, A61K 36/74, A24B 15/30

(54) **Product comprising kratom**

(71) Applicant: Fly High Amsterdam B.V., 2152 LL Nieuw-Vennep (NL)
(72) Inventor: Beers, Marcel Robert, 2523 JS DEN HAAG (NL); Kotlewski, Jörg, 32289 RÖDINGHAUSEN (DE); Schnetkamp, Yves Harry Roland, 2152 LL NIEUW-VENNEP (NL)
(74) Representative: Geurts, Franciscus Antonius

(57) **Abstract**

The present invention relates to a non-tobacco product comprising kratom. The invention further relates to a combination of tobacco and said product and a smoking article for smoking said product or combination. The invention also relates to a method for producing products of the invention.

## Description

### Field of the invention

The present invention relates to a non-tobacco product comprising kratom. The invention further relates to a combination of tobacco and said product and a smoking article for smoking said product or combination. The invention also relates to a method for producing products of the invention.

### Background of the invention

The kratom tree (*Mitragyna speciosa*) is a tropical tree from the coffee family (Rubiaceae). *Mitragyna speciosa* naturally grows in the area of Southeast Asia in the Malesia and Indochina floristic regions. The term "kratom" in relation to the invention is meant to refer to material derived from the *Mitragyna speciosa* plant, in particular leaves.

The leaves are known to be used as medicinal products. In areas where kratom trees naturally grow, kratom is usually chewed, in raw leaf form. Traditional use of kratom is considered equivalent to drinking coffee. *M. speciosa* material is regularly mixed or co-ingested with dextromethorphan-containing cough syrup, amphetamines, or benzodiazepines.

Kratom and derivatives thereof are also known as a substitute for opium drugs and as an aid for the management of opiate withdrawal.

In Thailand, kratom is also therapeutically used as an antidiarrheal, as a treatment for opioid dependence, and sometimes to increase the duration of coitus.

Kratom may also be smoked. It is to be understood that for purposes of the invention the term "smoking" or the verb "to smoke" is meant to refer to its meaning of drawing in and exhaling smoke from for example a cigarette, a e-cigarette of a waterpipe by a user. In case kratom is smoked, it will give the user a relaxing experience. How-ever, large amounts of leaves need to be smoked before the user notices the first small effects, while the duration of the relaxing experience is rather short (in the order of half an hour).

Another disadvantage of smoking kratom is that most smokers experience the smell of burned kratom leaves as unpleasant and sometimes disgusting. This smell occurs when kratom is smoked as pure kratom leaves, but also when kratom is smoked in combination with tobacco.

With the present invention the inventors have sought to resolve the negative effects involved with smoking kratom leaves.

The inventors have found that a product comprising concentrated kratom extract and concentrated aromas can be used in smoking kratom without the negative effects experienced with smoking kratom leaves.

### Description of the invention

In one aspect the invention relates to a non-tobacco product comprising a concentrated extract of kratom leaves and an aroma composition comprising an aroma conferring substance.

The term "non-tobacco product" refers to a product that is free of tobacco. The product of the invention may be regarded as a tobacco additive or a tobacco replacement. The composition may be smoked in combination with tobacco (cut tobacco) or without tobacco depending on the wishes of the user. Thus, in a further aspect the invention relates also to the combination of tobacco and the non-tobacco product of the invention, in particular the non-tobacco product suitable for use in a waterpipe or as a tobacco additive in a cigarette. Such combination can be regarded as a product obtained by combining the product of the invention with tobacco. The tobacco may either be cut tobacco such as shisha tobacco or regular tobacco or snuff tobacco, or a combination thereof. The invention also relates to a smoking article comprising the product or a combination of the product of the invention and tobacco, such as a waterpipe, a cigarette or an electronic cigarette. The product or combination of the invention, when smoked, gives the smoker a longer and more profound relaxing experience while only a small amount of burned material is inhaled, because a concentrated kratom extract is used. Therefore the user smoking the composition will inhale less of the harmful materials involved with smoking while the effects of the kratom are more intense and longer lasting. In addition, the composition of the invention does not produce the bad smells that occur when smoking kratom leaves.

In the product of the invention a concentrated kratom extract is used. This concentrated extract may be in the form of a powder or a liquid, such as a kratom suspension, solution or tincture. Kratom powder may be dissolved in in alcohols, such as ethanol, isopropanol, 2-propanol, propylene glycol, glycerol etc. Preferably, a kratom solution is made by dissolving dried kratom powder in ethanol. Suitable extracts for the purposes of the invention are extracts that are 15 x, 50 x or 80 x concentrated and are commercially available. 15 x 50 x or 80 x concentrated means that 1 gram of extract is extracted from 15, 50 or 80 grams of dried kratom leaves respectively. For the product of the invention, preferably concentrates that are more than 15 x concentrated are applied, because this will improve the user's smoking experience.

The concentrated kratom extract may as well be used in the form of a resin. The resin is formed by boiling down kratom leaves in water until a thick, sticky substance sticky substance remains. This resin extract is malleable and soft because it is boiled at a low temperature. One gram resin usually contains the active substances of about 7 grams of fresh kratom leaves.

In a non-tobacco product of the invention one or more aroma compositions may be used. An aroma composition used in the invention may be any suitable aroma comprising flavour or odour conferring substances. It is therefore to be understood that the term "aroma" comprises flavourings as well as odorants. The aromas in the composition may be added into the composition in solid (e.g. powder form) or in liquid form. Liquid aromas are preferred. Particularly suitable aroma compositions are so called e-liquids or e-juices. E-liquids are well known in the field of electronic cigarettes and various e-liquids are commercially available. An e-liquid is a solution used in e-cigarettes, which when heated via an atomizer, becomes a vapor, which the user inhales. An e-liquid solution may vary in its composition. Some e-liquids use propylene glycol as their base, while others use vegetable glycerol or a mixture thereof. Other variants may have a different base, for instance any suitable alcohol such as ethanol, isopropanol, 2-propanol or a mixture thereof. E-liquids may contain one aroma conferring substance or a combination of aroma conferring substances.

Suitable e-liquids contain approximately 5 vol.% of an aroma conferring substance. A non-limiting example of an e-liquid may be composed of 65,3-66,5 vol.% propylene glycol, 28-28,5% vol.% glycerol and 5 vol. % of an aroma conferring substance. Optionally, the e-liquid may contain nicotine, preferably up to 1,8 weight % nicotine, such as 0,6, 1,2 or 1,8 weight %. Other e-liquids may contain propylene glycol and glycerol in another volume/volume ratio, for instance approximately 1:1. E-liquids may as well contain ethanol.

Any desired aroma conferring substance may be incorporated in the aroma composition or e-liquid. In particular water or alcohol soluble foodstuff flavourings may be used for incorporation in the aroma. A non-limiting list of examples of suitable aroma conferring substances includes ethyl acetate, isoamyl acetate, propyl isobutyrate, ethyl butyrate, ethyl valerate, benzyl formate, menthol limonene, cymene, pinene, linalool, geraniol, citronellol, citral, peppermint oil, orange oil, lemon oil, borneol, tobacco extract, cocoa extract, licorice extract, and fruit extract or any combination of two or more thereof.

The amount of aroma composition comprised in the product of the inventions may differ depending on the wishes of the user and the nature of the composition. Also the concentration of the aroma conferring substance in such a composition will be an important parameter for deciding the amount of composition to be incorporated in the product of the invention. It is to be understood that when percentages are used to define embodiments of the product of the invention, the sum of all components comprised in said product is 100%.

Additional components may be combined with the product of the invention. For instance snuff tobacco may be added in an amount of for example 2 weight %. Snuff tobacco of the Moroccan variant is in particular suitable. The addition of snuff tobacco increases alertness of the user. For the same purpose caffeine may be added.

Also moxa may be added. Moxa is made from dried mugwort and adds an extra soothing experience to the user smoking the product of the invention. Preferably, Chinese moxa is used. A typical amount would be between 15 and 25 weight %, such as 20 weight% of moxa powder based on the weight of the product.

Depending on the wishes of the users the product of the invention may also comprise nicotine. Typical concentrations of nicotine may be between 0 and 0,18 weight % based on the weight of the product, such as 0,06, 0,12 or 0,18 weight %.

Colorants may also be added. Colorants may for instance be added to improve the appearance of product. For instance henna may be used in a typical percentage of approximately 5 weight%.

Additional additives comprise sugars, sweeteners and binding agents. Preferable binding agents include citrus fibers and gum arabic. Citrus fibers are in particular preferred.

In a further embodiment of the invention the non-tobacco product according to the invention comprises in addition to a concentrated extract of kratom leaves and at least one aroma composition comprising an aroma conferring substance also at least one of glycerol, propylene glycol and ethylene glycol. This product is in particular suitable for use in a waterpipe. For purposes of the invention it is preferred to use vegetable glycerol.

The term "waterpipe" is meant to relate to a single- or multi-stemmed instrument for vaporizing and smoking tobacco in which the vapor or smoke is passed through a water basin (often glass-based) before inhalation. Equivalent terms for a waterpipe are calean, chicha, hookah, hubble-bubble, hubbly-bubbly, kalian, nargileh, sheesha or shisha. Depending on the placement of the coal above the shisha tobacco, a waterpipe can be used to produce smoke by burning the shisha tobacco or to create water vapor by melting it at a lower temperature. According to the invention the product of this embodiment may be combined in a waterpipe with shisha tobacco. The invention therefore also relates to a waterpipe comprising a combination of tobacco (such as cut tobacco and/or snuff tobacco) and the product of this embodiment.

A non-tobacco product suitable for use in a waterpipe typically comprises:
a concentrated extract of kratom leaves in an amount that is equivalent to 0,15 to 750 grams of dried kratom leaves per 100 ml of product;
an e-liquid in an amount of 0,1 to 95 vol.% based on the volume of the product;
at least one of glycerol, propylene glycol and ethylene glycol in a total amount of at least 1 vol.%., such as at least 5 or 10 %, based on the volume of the product. In this product glycerol may be the basis. It is also possible to use propylene glycol or a mixture of propylene glycol and glycerol as a basis in an amount of at least 1 vol.%, such as at least 5 or 10 %. Although less preferred, any other alcohol, such as ethanol, isopropanol, 2-propanol, may be used as a basis for this non-tobacco product. When the non-tobacco product is used for smoking it is experienced that using glycerol, propylene glycol or a mixture of propylene glycol and glycerol as a basis, gives the most agreeable smoking experience, because the inhaled smoke gives a smooth feeling.

To produce this product, for example the following components may be mixed: liquid kratom extract (15x) in a percentage of 0,01- 50 vol.%; a liquid aroma in a total percentage of 0,1- 95 vol.%; the basis being glycerol, wherein the vol.% of the glycerol is at least 5%. The skilled person will acknowledge that various approaches are possible that may result in this product, in particular depending on the composition of the kratom extract and the aroma.

An exemplary product typical for this embodiment may contain 4 vol.% of an e-liquid, wherein the e-liquid is composed of 65,3-66,5 vol. % propylene glycol, 28-28,5 vol. % glycerol and 5 vol. % of an aroma conferring substance; 1 - 10 vol.% of a liquid 15x kratom extract while the remainder is glycerol. Such a product will contain the equivalent of 15 grams - 150 grams of dried kratom leaves per 100 ml of product.

In another embodiment the non-tobacco product according to the invention comprises in addition to a concentrated extract of kratom leaves and at least one aroma composition comprising an aroma conferring substance also ethanol and at least one of glycerol, propylene glycol and ethylene glycol. This product is in particular suitable for use in an electronic cigarette.

The term "electronic cigarette" (or e-cigarette), or personal vaporizer (PV), or electronic nicotine delivery system (ENDS) is meant to refer to an electronic inhaler which is meant to simulate and substitute for tobacco smoking. An electronic cigarette generally utilizes a heating element that vaporizes a liquid solution.

The invention also relates an electronic cigarette comprising the product of this embodiment.

A non-tobacco product typically suitable for use in an electronic cigarette preferably comprises:
ethanol in a percentage of 70-90 vol.% based on the volume of the product;
at least one of glycerol, propylene glycol and ethylene glycol in a total percentage of at least 1 vol.% based on the volume of the product,
an e-liquid in an amount of 0,1 to 10 vol.% based on the volume of the product, and
a concentrated extract of kratom leaves in an amount that is equivalent to 0,15 to 750 grams of dried kratom leaves per 100 ml of product.

In a more preferred embodiment glycerol is present in a percentage of between 6,5 and 8,5 vol.%, more preferably 7 or 8 vol.%.

For example a liquid for an electronic cigarette according to the invention may contain 80 vol.% ethanol, 5 vol.% glycerol, 5 vol.% of e-liquid (the e-liquid being composed of 65,3-66,5 vol. % propylene glycol, 28-28,5 vol. % glycerol and 5 vol. % of a flavour conferring substance or aroma), and 10 vol.% of kratom extract. It is estimated that such a product contains the equivalent of 150 grams of dried kratom leaves per 100 ml of product.

In another embodiment the non-tobacco product according to the invention relates to a product comprising at least sugar, at least one aroma composition comprising an aroma conferring substance, vegetable oil, at least one binding agent and a concentrated extract of kratom leaves. This product is in particular suitable for use as a tobacco additive. As sugar preferably regular granulated food grade sugar is used. The aroma composition used is preferably an e-liquid. Preferred binding agents used in this embodiment include citric fibers and gum arabic. Citrus fibers are particularly preferred, preferably in an amount of approximately 10 weight % based on the total weight of the combined components. The kratom extract used in this embodiment is preferably in the form of a resin.

The product of this embodiment is typically solid or semi-solid as an end-product and its appearance may be similar to hashish. It may be used in a similar way as hashish in a cigarette in combination with conventional cut tobacco.

In this respect the invention also relates to a cigarette comprising a combination of cut tobacco and the product of this embodiment.

The non-tobacco product of this embodiment typically comprises at least the components in the following amounts based on the total weight of the combined components:
sugar: 50-70 weight %;
e-liquid: 0,1 to 10 vol.%,
vegetable oil: 1-5 weight %;
citrus fibers: 5-20 weight %; and
a concentrated extract of kratom leaves in an amount that is equivalent to 1,5 to 150 grams of dried kratom leaves per 100 grams of product. A percentage of citrus fibers between 8 and 10 weight % is preferred.

The product of this embodiment is in particular suitable for combination with snuff tobacco, preferably wherein the snuff tobacco is distributed throughout the product of this embodiment. In this respect the invention relates also to combination of the product of this embodiment and snuff tobacco, wherein the snuff tobacco is distributed throughout the product.

For example a combination of a product of this embodiment with snuff tobacco may be obtainable by combining 60 weight % of granulated sugar, 20 weight % of moxa powder, 5 weight % of an e-liquid, 2 weight % vegetable oil, preferably hemp oil or hempseed oil, 5 weight % henna, 10 weight % of citrus fibers and 8 weight of snuff tobacco and a concentrated extract of kratom leaves in an amount that is equivalent to 15 grams of dried kratom leaves per 100 grams of product.

The invention relates also to a method for producing the product according this embodiment. This method comprises the following steps:
a)heating granulated sugar until boiling;
b)adding under agitation at least one aroma composition comprising an aroma conferring substance, vegetable oil, at least one binding agent, and a concentrated extract of kratom leaves;
c) maintaining boiling and agitation for a maximum of 5 minutes; and
d) cooling down the mixture until a semi-solid product is obtained.

In a preferred embodiment of this method, first sugar in an amount of 50-70 weight % based heated until boiling, followed by addition of e-liquid in an amount of 0,1 to 10 vol.%, vegetable oil in an amount of 1-5 weight %; citrus fibers in an amount of 5-20 weight%, all percentages based on the total weight of the combined components; and a concentrated extract of kratom leaves in an amount that is equivalent to 1,5 to 150 grams of dried kratom leaves per 100 grams of product. This mixture is then boiled and agitated for a maximum of 5 minutes followed by cooling down the mixture until a semi-solid product is obtained.

The concentrated extract of kratom leaves in this method may be in the form of powder or resin. The extract is preferably in the form of a kratom resin because the resin will easily melt in the heated sugar, which results in an end product with more homogenously dispersed kratom. The end product prepared with resin will also have a better appearance than when kratom powder would have been used. The resin is preferably used in an amount of 1-30 weight % based on the weight of the product.

In a more preferred embodiment this method comprises in addition a step of e) pressing the semi-solid product of step d). By pressing the semi-solid product air bubbles are removed, making the product more compact and giving it a more attractive appearance.

For example a product the invention comprising sugar, at least one aroma composition comprising an aroma conferring substance, vegetable oil, and a concentrated extract of kratom leaves in combination with snuff tobacco may be obtainable by combining 200 g of granulated sugar, 30 g of a 15, 50 or 80 x concentrated extract of kratom leaves, 100 g of moxa powder, 5 ml of an e-liquid, 5 ml hemp oil, 2 g henna, 10 ml of a blue colorant, 30 g citrus fibers and 30 g snuff tobacco. For this purpose sugar is heated until it boils. Subsequently moxa powder, the liquid aroma composition, the vegetable oil, the snuff, and the henna are added under agitation or stirring. The mixture is left to boil for a maximum of 5 minutes and subsequently left to cool down. The cooled down material is rolled to obtain a flat sheet of material and pressed to remove air bubbles. The sheet may be cut into blocks of appropriate size for further purposes.

## Claims

1. A non-tobacco product comprising a concentrated extract of kratom leaves and an aroma composition comprising an aroma conferring substance.

2. The non-tobacco product according to claim 1, wherein said aroma composition is an e-liquid.

3. The non-tobacco product according to claim 1 or 2, which further comprises at least one of glycerol, propylene glycol and ethylene glycol.

4. The non-tobacco product according to claim 3, wherein the product comprises:
a concentrated extract of kratom leaves in an amount that is equivalent to 0,15 to 750 grams of dried kratom leaves per 100 ml of product;
an e-liquid in an amount of 0,1 to 95 vol.% based on the volume of the product;
at least one of glycerol, propylene glycol and ethylene glycol in a total amount of at least 1 vol.% based on the volume of the product.

5. The non-tobacco product according to claim 3, which also comprises ethanol.

6. The non-tobacco product according to claim 5, wherein the product comprises:
ethanol in a percentage of 70-90 vol.% based on the volume of the product;
at least one of glycerol, propylene glycol and ethylene glycol in a total percentage of at least 1 vol.% based on the volume of the product,
an e-liquid in an amount of 0,1 to 10 vol.% based on the volume of the product, and
a concentrated extract of kratom leaves in an amount that is equivalent to 0,15 to 750 grams of dried kratom leaves per 100 ml of product.

7. The non-tobacco product according to claim 1 or 2, comprising at least the following components: sugar, at least one aroma composition comprising an aroma conferring substance, vegetable oil, at least one binding agent and a concentrated extract of kratom leaves.

8. The non-tobacco product according to claim 7, comprising at least the components in the following amounts based on the total weight of the combined components:
sugar: 50-70 weight %;
e-liquid: 0,1 to 10 vol.%,
vegetable oil: 1-5 weight %;
citrus fibers: 5-20 weight %; and
a concentrated extract of kratom leaves in an amount that is equivalent to 1,5 to 150 grams of dried kratom leaves per 100 grams of product.

9. Method for producing the product according to claim 7 or 8, comprising the following steps:
a)heating granulated sugar until boiling;
b)adding under agitation at least one aroma composition comprising an aroma conferring substance, vegetable oil, at least one binding agent and a concentrated extract of kratom leaves;
c) maintaining boiling and agitation for a maximum of 5 minutes; and
d) cooling down the mixture until a semi-solid product is obtained.

10. Method according to claim 9, which comprises in addition a step of e) pressing the semi-solid product of step d).

11. Combination of tobacco and the product defined in any of the claims 1-4 and 7-8.

12. Combination according to claim 11, wherein said tobacco is cut tobacco and/or snuff tobacco.

13. Combination according to claim 11, wherein said product is the product defined in claims 7 or 8, and wherein said tobacco is snuff tobacco distributed throughout the product defined in claims 7 or 8.

14. Smoking article selected from the group of:
a) a waterpipe comprising the combination of claim 11 or 12;
b) a cigarette comprising the combination of any of the claims 11-13; and
c) an electronic cigarette comprising the product of claims 5 or 6.
